# EUROPEAN PATENT APPLICATION

(11) **EP 4 796 105 A1**
(43) Date of publication of application: **26.08.2026**
(21) Application number: 25305247.6
(22) Date of filing: 25.02.2025
(51) Int. Cl.: A61K 9/00, A61K 31/00, A61K 47/10, A61P 25/04, A61P 25/30, A61P 29/00, A61K 9/72, A24F 40/10, A61M 11/04, A61M 15/06

(54) **LIQUID AND VAPORIZABLE COMPOSITION COMPRISING CANNABINOID(S) FOR THERAPEUTIC USE**

(71) Applicant: Versevrage, 38120 Fontanil-Cornillon (FR)
(72) Inventor: OMBÉ, Sébastien, 38500 Voiron (FR)
(74) Representative: Icosa

(57) **Abstract**

The present invention relates to a liquid and vaporizable composition comprising:
(1) a distillate of at least one cannabinoid selected from THC, CBD and their mixtures thereof wherein the distillate comprises less than 1% by weight of terpene, based on the total weight of distillate,
(2) and at least one diol selected from 1,2-propanediol 1,3-propanediol, 1,3-butanediol, 1,4-butanediol, 2-methyl-1,3-propanediol, polyethylene glycol (PEG), polyethylene glycol 400 (PEG 400), and mixtures thereof.

The composition is used as a medicament wherein a constant dose of cannabinoid(s) is delivered to a subject in need through a vaporizing device.

## Description

### FIELD OF INVENTION

The present invention relates to a liquid and vaporizable composition comprising cannabinoid(s) for use as a medicament. It also relates to a vape device that delivers the said composition.

### BACKGROUND OF INVENTION

Cannabinoids, the active compounds found in the cannabis plant, have emerged as a significant area of interest in medical research and therapeutics. These compounds interact with the body's endocannabinoid system (ECS), a complex cell-signaling network that plays a crucial role in regulating physiological processes such as pain, mood, appetite, and immune responses. As the understanding of cannabinoids deepens, their potential to treat a wide range of medical conditions is becoming increasingly evident.

Among the numerous cannabinoids identified in cannabis, a few stand out for their established and potential therapeutic applications.

Delta-9-Tetrahydrocannabinol (THC) is the most well-known cannabinoid due to its psychoactive effects. THC interacts directly with CB1 and CB2 receptors in the ECS, producing a range of physiological and psychological effects. Medically, THC has proven effective in managing chronic pain, particularly neuropathic pain, and in addressing chemotherapy-induced nausea and vomiting. It is also used to stimulate appetite in conditions such as HIV/AIDS-related cachexia. The U.S. Food and Drug Administration (FDA) has approved medications like dronabinol and nabilone that leverage THC for these purposes.

In contrast, Cannabidiol (CBD) is non-psychoactive and has gained attention for its broad therapeutic potential. CBD's ability to modulate ECS activity without directly binding to CB1 or CB2 receptors makes it an attractive option for conditions such as epilepsy, anxiety, and inflammation. The FDA-approved medication Epidiolex, derived from CBD, has revolutionized the treatment of severe epilepsy syndromes like Dravet syndrome and Lennox-Gastaut syndrome. Moreover, CBD shows promise in managing chronic pain, autoimmune diseases, and post-traumatic stress disorder (PTSD), offering a safer alternative to traditional medications.

Other cannabinoids, though less well-known, are also being explored for their medicinal benefits. Cannabigerol (CBG), a precursor to THC and CBD, has demonstrated potential in treating glaucoma, inflammatory bowel disease, and bacterial infections. Similarly, Cannabinol (CBN), a mildly psychoactive compound formed as THC degrades, is being studied for its sedative properties and potential as a sleep aid. Tetrahydrocannabivarin (THCV), another cannabinoid, shows promise in regulating blood sugar levels in diabetes, suppressing appetite, and possibly aiding neurological conditions like Parkinson's disease.

The delivery of cannabinoids varies depending on the condition being treated and the desired onset of effects. Common methods include oral consumption through pills, oils, and edibles, which provide systemic effects over a prolonged period. Topical applications, such as creams and patches, are effective for localized pain or skin conditions, while sublingual tinctures allow for quick absorption without the need for inhalation. Inhalation, via smoking or vaping, offers rapid relief and is often used for acute symptoms.

Despite the therapeutic potential of cannabinoids, their efficacy is often limited by the challenges associated with delivery methods. The method of administration significantly affects the onset, duration, and intensity of therapeutic effects, as well as patient compliance and safety. Current delivery systems face several issues that hinder the optimal use of cannabinoids in medical treatments.

Inhalation, including smoking and vaping, is a popular method for cannabinoid delivery due to its rapid onset of effects. Cannabinoids delivered through inhalation are absorbed directly into the bloodstream via the lungs, bypassing the digestive system and first-pass metabolism. However, this method comes with several significant drawbacks related to health risks, dosing inconsistencies.

The primary concern with smoking cannabis is the inhalation of harmful byproducts produced during combustion. When cannabis is smoked, it generates tar, carbon monoxide, and other carcinogens, which can cause respiratory damage over time. This is particularly concerning for medical users who may already have compromised health.

Regular smoking of cannabis has been associated with symptoms such as chronic cough, wheezing, and phlegm production, indicative of bronchial irritation.

Cannabis smoke contains carcinogens and exposes users to potentially harmful compounds that can increase the risk of lung diseases.

Vaping, considered a safer alternative, also has its challenges. In 2019, a wave of vaping-associated lung injury (VALI) cases highlighted the risks of vaporized oils. Contaminants, including vitamin E acetate and other additives, were identified as major culprits. Plant oils are also found to be dangerous for inhalation, as they cause pulmonary embolisms. Even with safer formulations, long-term effects of vaping are not fully understood.

While inhalation offers undeniable advantages, particularly its rapid onset of effects, it also presents a range of significant challenges. These include respiratory health risks and inconsistent dosing, which can compromise its suitability as a medical delivery method. Continued research into safer alternatives, better patient education, and technological innovations in addressing these issues and ensuring that inhalation remains a viable option for cannabinoid-based therapy is still essential.

Therefore, it remains necessary to develop a composition and/or a device that will allow the control of active ingredients dosage and their delivery to a subject in need thereof. To control the active ingredients dosage via inhalation, it is necessary to develop a composition and/or a device that will ensure the constant delivery of the active ingredients.

More specifically, it is essential to develop a secure and effective system for delivering cannabinoids to a subject for therapeutic use.

Surprisingly, the inventors have found that a cannabinoid formulation that comprises diols as solvent will permit a better dissolution of the active ingredients without the addition of any other component, that will ensure a significantly lower viscosity of the formulation compared to a formulation without the added solvent, which will help a better vaporization of the cannabinoids and then a better bioavailability of the active ingredients by inhalation. Additionally, purer formulation ensures greater safety in its composition but also during the use of a vaporizing device since the formation of degradation products will be minimal. Furthermore, the inventors have also found that the diols that have a close ebullition temperature compared to the ebullition temperature of cannabinoids are favorable for the implementation of the composition into a vaporizing device that will heat the ingredients to their boiling temperatures and thus aerosolize the composition including the active ingredients to ensure their delivery deeply into the lungs. A fluid formulation will lead to a more homogeneous and efficient vaporization with lower temperatures. This also contributes to improved bioavailability of the active ingredients.

### SUMMARY

The present invention thus relates to a liquid and vaporizable composition comprising:
(1) a distillate of at least one cannabinoid selected from THC, CBD and their mixtures thereof,
   wherein said distillate comprises less than 1% by weight of terpene, based on the total weight of distillate, and
(2) at least one diol selected from 1,2-propanediol 1,3-propanediol, 1,3-butanediol, 1,4-butanediol, 2-methyl-1,3-propanediol, polyethylene glycol (PEG), polyethylene glycol 400 (PEG 400), and mixtures thereof; the content of diols ranging from 80 to 98,5% by weight, based on the total weight of the composition,
for use as a medicament, wherein a constant dose of cannabinoid(s), ranging from 50 µg to 700 µg per puff and/or per breathable dose, is delivered to a subject in need through a vaporizing device.

The present invention also relates to a kit comprising:
(a) a liquid and vaporizable composition comprising
   (1) a distillate of at least one cannabinoid selected from THC, CBD and their mixtures thereof, wherein said distillate comprises less than 1% by weight of terpene, based on the total weight of distillate, and
   (2) at least one diol selected from 1,2-propanediol 1,3-propanediol, 1,3-butanediol, 1,4-butanediol, 2-methyl-1,3-propanediol, polyethylene glycol (PEG), polyethylene glycol 400 (PEG 400), and mixture thereof; the content of diols ranging from 80 to 98,5% by weight, based on the total weight of the composition; and
(b) vape device able to deliver a constant dose of cannabinoid(s), ranging from 50 µg to 700 µg per puff and/or per breathable dose.

The present invention further relates to a vape device comprising the liquid and vaporizable composition of the present invention to deliver a constant dose of cannabinoid(s), ranging from 50 µg to 700 µg per puff and/or per breathable dose.

### DEFINITIONS

In the present invention, the following terms have the following meanings:
"distillate" refers to the purified liquid or concentrate obtained through the process of distillation, where a mixture is heated to separate its components based on differences in their boiling points. The vapor produced is then condensed back into a liquid form.
**"Terpene"** refers to a class of natural products consisting of compounds with the formula (C₅H₈)ₙ for n ≥ 2.
**"Diol",** is a type of organic compound that contains two hydroxyl groups (-OH) attached to carbon atoms in its molecular structure.
"**A puff**" refers to a single actuation of an inhaler, releasing a pre-measured amount of medication, usually an aerosol or dry powder, into the mouthpiece.
**"A nominal dose"** refers to the total amount of a drug released or delivered from a medical device, such as an inhaler, before any losses occur during administration. It is the labelled dose or the dose stated by the manufacturer for a single actuation, i.e. a puff, of the device.
**"A breathable dose"** or **"respirable dose"** refer to the fraction of the medication from the puff that is actually inhaled into the lungs and deposited on the respiratory surfaces where it can exert its therapeutic effect. It is influenced by factors such as particle size, inhalation technique, and the patient's breathing pattern. Not all of the nominal dose from the puff becomes a breathable dose due to drug deposition in the mouth or throat (oropharyngeal deposition). The breathable dose is the effective dose. The Mass Median Aerodynamic Diameter (MAD) and Fine Particle Fraction (FPF) are key parameters in determining the fraction of inhaled particles that reach the deep lung. According to the invention, a breathable dose preferably refers to when aerosolized cannabinoids have a particle size fraction with a diameter of less than 10 µm, more preferably less than 8 µm, advantageously less than 6,4 µm, even more preferably less than 2 µm.
**"Granulometry"** refers to the study of particle sizes within a sample. In inhalation studies, granulometry helps determine aerosol particle size distribution, which influences deposition in different parts of the respiratory system. Typical methods include laser diffraction, cascade impaction, and sieving. Particles under 7 µm are considered respirable, while larger ones tend to deposit in the upper airways.
**"A vaporizing device"** refers to a device designed to heat a liquid into an aerosol that the user inhales. The terms "vaporizing device" also refers to vaping device, electronic cigarette, cigalikes, pod systems, vape pens, box mods, mechanical mods, disposable vapes and desktop vaporizers.
**"atomizer"** refers to a component consisting of an atomizer head connected to the e-liquid tank.
**"Puff activator"** refers to a function required to start the aerosol generation process in the product under test
**"Aerosol"** refers to a system of colloidal particles suspended in the gas generated using an electronic cigarette.
**"Cartridge"** refers to a disposable or refillable e-liquid container designed to be inserted into an electronic cigarette.
**"Electronic cigarette"** refers to a device for transforming e-liquid into an aerosol, produced by the passage of air through the device and intended for inhalation.
**"Steaming time"** refers to the time during which the atomizer operates and produces steam without interruption.
**"Puff duration"** refers to the time interval, measured in seconds, during which the machine port is pneumatically connected to the suction mechanism.
**"e-liquid"** refers to a liquid containing or not nicotine, intended to be completely transformed into an aerosol by an electronic cigarette.
**"Constant CBD/THC emission"** refers to the emission in which the CBD/THC concentration measured over a series of puffs is contained within a predefined range.
**"Puff period"** refers to the time between the start of one puff and the start of the next puff. In an embodiment, the puff period is between 2 and 6 seconds, preferably 3 seconds with a flow rate of at least 1,1L/min, (AFNOR (2021) Cigarettes électroniques et e-liquides - Exigences et méthodes d'essai relatives aux émissions (XP D 90-300-1). AFNOR), preferably at least 2L/min. Advantageously, the flow rate is between 1,1L/min and 10L/min, even more preferably is 2L/min.
**"Puff profile"** refers to the air flow rate measured over the duration of a puff, usually plotted against time.
**"Target value"** refers to the value calculated for a defined number of puffs per day. It is to be compared with the value measured for emissions.
**"Puff volume"** refers to the volume inhaled by the vaping machine at the tip of the electronic cigarette mouthpiece.
**"treatment", "treat"** or **"treating"** refer to any act intended to ameliorate the health status of patients such as therapy, prevention, prophylaxis and retardation of a disease. In certain embodiments, such terms refer to the amelioration or eradication of the disease, or symptoms associated with it. In other embodiments, this term refers to minimizing the spread or worsening of the disease, resulting from the administration of one or more therapeutic agents to a subject with such a disease. The term **"treatment"** or **"treating"** also refers to delaying the onset of a disease or disorder. In this context, the term **"prevention"** refers to a reduction in the risk of acquiring a specified disease or disorder.
**"subject", "individual"** or **"patient"** are interchangeable and refer to a human, including adult, child, newborn or human at the prenatal stage.
**"quantity", "amount",** and **"dose"** are used interchangeably herein and may refer to an absolute quantification of a molecule.
**"active principle", "active ingredient"** and **"active pharmaceutical ingredient"** are equivalent and refer to a component of a pharmaceutical composition having a therapeutic effect.
**"therapeutic effect"** refers to an effect induced by an active ingredient, or a pharmaceutical composition according to the invention, capable to prevent or to delay the appearance of a disease, or to cure or to attenuate the effects of a disease.
**"Consisting of**" or **"consist"** is to be construed in a close, non-inclusive sense, limited to the features following this term.
**"Comprising"** or **"comprise"** is to be construed in an open, inclusive sense, but not limited to.

In this description, unless otherwise specified, it is understood that, when an interval is given, it includes the upper and lower bounds of said interval.

### DETAILED DESCRIPTION

This invention relates to a liquid and vaporizable composition comprising:
(1) a distillate of at least one cannabinoid selected from THC, CBD and their mixtures thereof,
   wherein said distillate comprises less than 1% by weight of terpene, based on the total weight of distillate, and
(2) a diol selected from 1,2-propanediol 1,3-propanediol, 1,3-butanediol, 1,4-butanediol, 2-methyl-1,3-propanediol, polyethylene glycol (PEG), polyethylene glycol 400 (PEG 400), and combinations thereof; the content of diols ranging from 80 to 98,5% by weight, based on the total weight of the composition,
for use as a medicament, wherein a constant dose of cannabinoid(s), ranging from 50 µg to 700 µg per puff and/or per breathable dose, is delivered to a subject in need through a vaporizing device.

### Distillate (1):

*Cannabis sativa* is a plant species belonging to the genus Cannabis, known for its wide range of uses that span recreational, medicinal, and industrial applications. As one of the three primary species in the cannabis family, alongside Cannabis indica and Cannabis ruderalis, it stands out for its unique characteristics and versatility. A key feature of *Cannabis sativa* lies in its chemical composition. The plant is rich in cannabinoids, chemical compounds that interact with the human body's endocannabinoid system. Among these, tetrahydrocannabinol (THC) and cannabidiol (CBD) are the most prominent. The plant also comprises terpenes.

However, the Applicant has found that to obtain a better vaporizable composition comprising cannabinoids, the composition needs to only contain traces of terpene, preferably to be terpene-free. According to the invention, "traces of terpene" and "terpene-free" refer to the composition wherein the distillate comprises less than 1% by weight of terpene.

According to the invention, the distillate comprises less than 1% by weight of terpene, based on the total weight of distillate. Advantageously, the distillate comprises less than 0,9% by weight of terpene, more preferably less than 0,5% by weight of terpene, even more preferably less than 0,1% by weight of terpene, better still less than 0.05% by weight of terpene, advantageously less than 0.01% by weight of terpene, more advantageously less than 0.005% by weight of terpene, better still less than 0.001% by weight of terpene, more preferably less than 0.0005% by weight of terpene and even more advantageously less than 0,0002% by weight of terpene, based on the total weight of distillate.

This invention first relates to a liquid and vaporizable composition comprising a distillate of at least one cannabinoid selected from THC, CBD and their mixtures thereof that is delivered to a subject in need through a vaporizing device.

The distillate (1) comprised in the composition of the present invention comprises at least one cannabinoid selected from THC, CBD and their mixtures thereof.

According to a first embodiment, the distillate (1) consists of THC or CBD.

According to a second embodiment, the distillate comprises a mixture of THC and CBD and the weight ratio THC:CBD ranges from 0.1 to 10, preferably from 0.5 to2, more preferably 0.1, 0.2, 0.3, 0.4, 0.5, 0.6, 0.7, 0.8, 0.9, 1, 2, 3, 4, 5, 6, 7, 8, 9 or 10.

In a preferred embodiment, the distillate comprises THC:CBD in a ratio of 1:0, i.e. it consists of THC.

In another preferred embodiment, the distillate comprises THC:CBD in a ratio of 0:1, i.e. it consist of CBD.

In another preferred embodiment, the distillate comprises THC:CBD in a ratio of 2:1, i.e. there is twice as much THC as CBD.

In another preferred embodiment, the distillate comprises THC:CBD in a ratio of 1:2, i.e. there is twice as much CBD as THC.

In another preferred embodiment, the distillate comprises THC:CBD in a ratio of 1:1, i.e. there is as much THC as CBD.

In another preferred embodiment, the distillate comprises THC:CBD in a ratio of 1:5, i.e. there is five times as much CBD as THC.

Advantageously, the content of cannabinoid(s), present in the distillate (1), ranges from 60 to 99% by weight, preferably from 80% to 95% by weight, based on the total weight of the distillate.

Advantageously, the content of distillate (1) ranges from 1,5% to 10% by weight, based on the total weight of the composition.

### The diol (2):

The composition according to the invention further comprises a diol (2) selected from 1,2-propanediol 1,3-propanediol, 1,3-butanediol, 1,4-butanediol, 2-methyl-1,3-propanediol, polyethylene glycol (PEG), polyethylene glycol 400 (PEG 400), and combinations thereof; the content of diols ranging from 80 to 98,5% by weight, based on the total weight of the composition.

Advantageously, the distillate according to the invention has a viscous consistency, with approximately a viscosity ranging from 5 000CP and 15 000cP. Therefore, it is important to dilute the distillate with the diol solvent to obtain a more fluid final composition. After dilution, the viscosity of the composition according to the invention preferably ranges from 50cP to 500cP, more preferably from 50CP to 300cP, even more preferably from 50cP to 200 cP, advantageously is 60cP.

The consistency of the composition according to the invention is appropriate for vaporizing devices that preferably comprise a mesh heater. Indeed, when the distillate is not diluted in the diol solvent, its consistency does not allow it to be inhaled with common vaporizing devices. For example, with a vaporizing device comprising a mesh heater, the viscous distillate may burn the mesh heater, while with a ceramic heater, a lot of degradation products, which are toxic, might be produced since high temperatures need to be reached to get smoke that can be inhaled. Thus, the breathable dose of the viscous distillate, when it is not diluted, is reduced. The dilution of the distillate in the specific diol solvents according to the present invention allows to get a less viscous composition.

CBD and THC are apolar molecules known as hydrophobic and lipophilic, not soluble in water and more soluble in fats. They need an apolar solvent to dissolve as well as possible, but they can also be dissolved via an organic solvent, such as propylene glycol. Indeed, the solubility of a compound such as CBD or THC in a solvent generally depends on the interactions that may exist between the solvent and said compounds.

The diols that are used according to the invention are highly fluid organic solvents that effectively dissolve CBD and THC, even at high concentrations of up to 1,500 mg per 10 ml. Notably, CBD and THC can be fully dissolved in a pure diol, according to the invention, base, without requiring any additional ingredients like vegetable glycerin and/or the addition of terpenes to thin the mixture that will lead to a bad vaporization of the composition and the formation of degradation products. Furthermore, the mixture obtained will lead to a better aerosolization of the active ingredients and thus to a better efficiency induced by a more important breathable dose, and by limiting the formation of degradation products due to complete vaporizable components.

Additionally, the diols selected for this invention ensure consistent vaporization and delivery of CBD and THC, as all three substances share a similar vaporization temperature of approximately 189°C, which ameliorates the bioavailability of the active ingredients. The not excessively high temperatures also contribute to avoiding aldehyde type degradation products, that can be toxic.
Here is a table summarizing the boiling points of the requested diols:

| **Compound** | **Boiling Point (°C)** |
|---|---|
| **1,2-Propanediol (Propylene Glycol)** | 188.2°C |
| **1,3-Propanediol** | 214°C |
| **1,3-Butanediol** | 207°C |
| **1,4-Butanediol** | 235°C |
| **2-Methyl-1,3-propanediol (Neopentyl Glycol)** | 211°C |
| **PEG** | 250°C |
| **PEG 400** | 250°C |

In a preferred embodiment, the boiling temperature of the diol is comprised between 170°C and 250°C, preferably between 180°C and 240°C.

This compatibility facilitates efficient and uniform release of CBD and THC, enhancing the reliability and efficacy of the product.

Advantageously, the diol is 1,3-propanediol. Indeed, 1,3-propanediol makes inhalation easier and without irritation.

The content of diols ranging from 80 to 98,5% by weight allows good vaporization of the active ingredients, which dilute even better in the 1,3-propanediol. In an advantageous embodiment, the content of diols ranges from 85 to 98,5 % by weight, more preferably from 90 to 98,5% by weight, based on the total weight of the composition.

### Composition preparation process

The distillate of the present invention is preferably obtained by the following process comprising the steps of:
a. grinding dried Cannabis sativa flowers, preferably dried Cannabis sativa flowers with a humidity level of 10%,
b. decarboxylation,
c. extraction, preferably with supercritical CO2, to obtain an extract,
d. molecular distillation of the extract to obtain a distillate, and
e. ethanol pre-treatment for filtration, preferably the distillate is dissolved in EtOH (extract/EtOH ratio 1/5 m/m), stored at -18°C, cold filtered and evaporated under vacuum to remove EtOH.

This process is favorable to obtain a final distillate with traces of terpene, preferably a terpene-free distillate. This process also permits to separate terpenes, chlorophyll and plant pigments from cannabinoids. This is achieved by the different, lower boiling temperatures of these components and the higher boiling temperatures of cannabinoids, which enable the cannabinoids to be separated from the other components, making the solution terpene-free.

Cannabinoids are naturally occurring compounds found in the cannabis plant, existing in two primary forms: acidic and neutral. The acidic forms, such as tetrahydrocannabinolic acid (THCa) and cannabidiolic acid (CBDa), are the precursors to their neutral counterparts, tetrahydrocannabinol (THC) and cannabidiol (CBD). While both forms have biological activity, the neutral versions are generally preferred due to their greater potency, bioavailability, and interaction with the human endocannabinoid system. Step b. of decarboxylation allows the conversion of cannabinoids from their acidic form to the neutral form. In another embodiment of the invention, the decarboxylation of step b. could also be carried out on the extract in the case of producing an extract from an undecarboxylated plant drug.

Regarding the molecular distillation of step d., depending on the target formulation and the nature of the soft extract, further distillation steps may be required to ensure extract solubility in the formulation vehicle and formulation stability.

Step e. could also be carried out prior to molecular distillation operations. It is optional and may be replaced by distillation steps, depending on the nature of the plant drug and the soft extract.

The distillate of the present invention may alternatively be obtained by the following process comprising the steps of:
a. a standardized THC extract at 75% is diluted in MCT oil (medium-chain triglycerides);
b. molecular distillation to remove the oil and keep the cannabinoids to obtain a distillate with high content of THC (80% by weight of the total w% of the distillate); and
c. ethanol pre-treatment for filtration, preferably the distillate is dissolved in EtOH (extract/EtOH ratio 1/5 m/m), stored at -18°C, cold filtered and evaporated under vacuum to remove EtOH.

According to another aspect, the invention preferably relates to a process of manufacturing of the composition comprising the steps of:
a. Preparation of the distillate (1), preferably according to one of the processes described previously,
b. Adding the diol (2) to the distillate,
c. Obtaining the composition according to the invention.
To obtain the composition according to the invention, the diol is added after the formation of the distillate.

The composition according to the invention may optionally further comprise an isolate of CBD in its crystalline or powder form. A CBD isolate is a pure form of cannabidiol (CBD), with a purity over 99%, that has been extracted from the cannabis plant and isolated from all other cannabinoids, terpenes, flavonoids, and plant compounds. In other words, an isolate contains only CBD.

The composition according to the invention, may also further comprise at least one acid, preferably lactic acid and/or benzoic acid, more preferably lactic acid. When they are present, the acids adjust the pH and help to stabilize the composition and avoid accelerated degradation of the cannabinoids.

### Therapeutic uses

The composition according to the invention is used as a medicament, wherein a constant dose of cannabinoid(s), ranging from 50 µg to 700 µg per puff and/or per breathable dose, is delivered to a subject in need through a vaporizing device.

According to an alternative embodiment, the invention relates to the use of the composition according to the invention for the manufacturing of a medicament, wherein a constant dose of cannabinoid(s), ranging from 50 µg to 700 µg per puff and/or per breathable dose, is delivered to a subject in need through a vaporizing device.

The cannabinoids are preferably delivered to the subject in need in a constant dose ranging from 55 µg to 500 µg per puff and/or per breathable dose, more preferably from 60 µg to 300 µg per puff and/or per breathable dose, even more preferably from 60 µg to 80 µg per puff and/or per breathable dose.

A constant dose refers to a fixed and unchanging amount of a substance or medication that is administered over a specific period of time. Unlike a variable or tapered dose, where the amount may be adjusted, a constant dose remains steady to maintain consistent levels of the substance in the body. According to the invention, the constant dose delivery is possible due to the formulation of the composition and the vaporizing device described below and is explained in the examples. More precisely, the constant dose according to the invention preferably refers to fixed values of the puff period and the flow rate. Therefore, the vaporized composition mass and the aerosolized active ingredients remain constant with repeatability. Combined with a restrained manufacturing deviation of the vaporizing device and the containers comprising the composition according to the invention, the variation of inter-puff and inter-container is minimal which leads to an inter-puff and inter-container repeatability.

According to an advantageous embodiment, the composition provides a subject in need with THC:CBD ranging from 50:0 to 10:50 mg/ml, preferably from 40:0 to 10:40 mg/ml.

According to one embodiment, the composition is used in a method of pain management. In a preferred embodiment, the composition is used in the prevention and/or treatment of chronic pain, painful spasticity, pain due to cancer, injury, accident, surgery, inflammation, tissue damage, arthritis, joint pain, pain from infection, gastrointestinal pain, diabetes, diabetes neuropathy, post-shingles neuralgia, neuropathic pain, peripheral neuropathy or multiple sclerosis, more preferably chronic pain or painful spasticity.

Chronic pain refers to a pain that persists or recurs for more than three months, beyond the usual course of acute illness or injury. It is a complex condition that can occur in the absence of an identifiable cause or be associated with an underlying health condition, such as arthritis, fibromyalgia, or nerve damage. Chronic pain can impact physical and emotional well-being, often interfering with daily activities, work, and quality of life.

Painful spasticity refers to a condition characterized by an abnormal increase in muscle stiffness or tone, accompanied by involuntary muscle contractions or spasms, which result in significant pain. Spasticity occurs due to dysfunction in the central nervous system, often involving damage to the brain or spinal cord, such as in conditions like multiple sclerosis, cerebral palsy, stroke, or spinal cord injury. The pain associated with spasticity can stem from the constant muscle tightness, spasms, or secondary complications like joint deformities and inflammation. Managing painful spasticity often requires a combination of medications, physical therapy, and sometimes surgical interventions.

Subjects in need of a therapeutic effect for pain management in the intended indications may use the formulation prior to, during, or after the medical event or need arises.

According to an alternative embodiment, the composition is used in the prevention and/or the treatment of cannabis addiction.

According to another alternative aspect, the invention relates to a method of treatment of cannabis addiction by inhaling the composition according to the invention using a vaporizing device.

Cannabis addiction, often referred to as cannabis use disorder, is a condition in which individuals develop a problematic and compulsive relationship with cannabis, despite experiencing negative consequences in various aspects of their lives. While cannabis is often perceived as a relatively benign substance, its potential for addiction has been increasingly recognized in both clinical settings and research. Cannabis use disorder is marked by several key characteristics, including compulsive use, tolerance, withdrawal symptoms, and significant impairment in daily functioning. Cannabis use disorder is recognized as a disease in clinical settings. The American Psychiatric Association (APA) includes it in the Diagnostic and Statistical Manual of Mental Disorders, Fifth Edition (DSM-5), which is the standard classification used by mental health professionals. According to the DSM-5, cannabis use disorder is classified as a substance use disorder-a medical condition that can lead to significant physical, psychological, and social consequences. In Europe, cannabis use disorder is also recognized as a medical condition, though its classification and approach to treatment can vary by country. The World Health Organization (WHO) and the European Monitoring Centre for Drugs and Drug Addiction (EMCDDA) both acknowledge the harmful potential of cannabis use, including the risk of developing cannabis use disorder. Cannabis dependence is often associated with nicotine dependence, due to the mixture of tobacco and dried flowers or resin, resulting in a codependence that is multifactorial, with a double physical dependence (THC and nicotine), behavioral and psychological dependence.

### The device

The composition according to the invention is delivered to a subject in need through a vaporizing device.

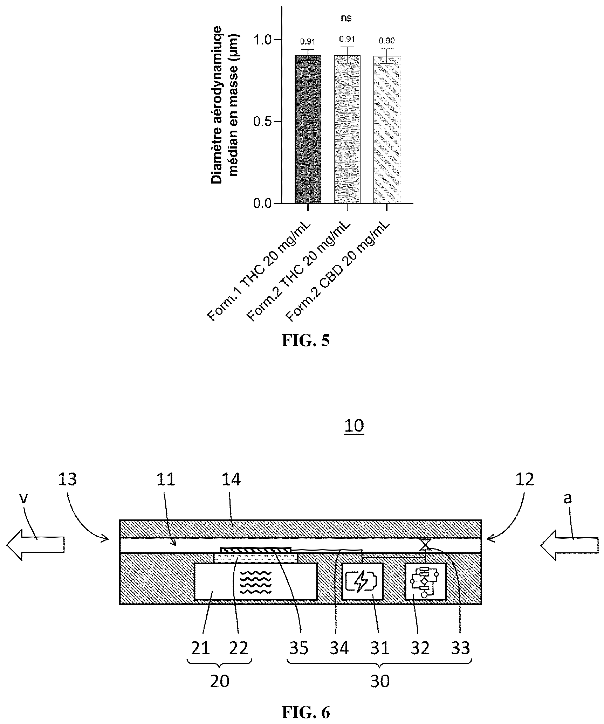

In reference to figure 6, a vaporizing device (10) comprises an e-liquid supply (20) configured to deliver an e-liquid, and an electronic system (30) configured to vaporize the delivered e-liquid into a duct (11), so that a user can breathe the vapor (v) from an outlet (13) of the duct (11). The e-liquid supply (20) and the electronic system (30) are held in a housing (14).

The e-liquid supply (20) comprises:
- a container (21) which contains the composition according to the invention;
- an absorbing barrier (22), which constitutes a barrier between the container (21) and the inside of the duct (11), and which plays a double role of:
   - avoid leakage of the e-liquid from the container (21); and
   - absorb e-liquid from the container (21) and drive the e-liquid, thanks to capillarity, in direction of the inside of the duct (11).

The electronic system (30) comprises the following devices:
- a power supply (31), configured to supply energy to the devices of the electronic system (30);
- a controller (32), configured to drive the devices of the electronic system (30), according to instructions stored into a readable memory of the controller (32);
- a heater (35), placed inside the duct (11) and in contact with the absorbing barrier (22), and configured to heat when it is energized by the power supply (31);
- optionally, a flow sensor (33), such as a flow-meter or a pressure sensor, configured to detect a measurement of an air flow (a) entering the duct (11) through an inlet (12); and
- wires (34) providing the necessary connections between devices of the electronic system (30).

When a user activates the vaporizing device (10), the controller (31) energizes the heater (35). Since the heater (35) is placed against the absorbing barrier (22) which is filled with e-liquid, the heat can be transferred from the heater (35) to the e-liquid which turns from liquid state to vapor state, thus generating the desired vaporized composition.

The aim of the vaporizing device is to deliver constant doses of the composition according to the invention. Several features help to reach this goal.

Firstly, the heater is preferably automatically manufactured, in order to guarantee the respect of strict manufacturing tolerances. Deviations relating to dimensions or shape of the heater (34) shall be as little as possible.

This way, it is easier to ensure that:
- the heater (34) of the vaporizing device (10) will behave as it was designed for;
- failures of the heater (34), caused by manufacturing mistakes, will be as reduced as possible;
- the heaters (34) of different vaporing devices (10) will behave in the same way, e.g. the same quantity of power supplied to each heater (34) generates the same amount of vapor of a predetermined e-liquid, no matter the device (10) from a manufacturing batch.

A restrained manufacturing deviation allows a high inter-puff repeatability no matter the formulation of the e-liquid composition, demonstrating an excellent performance on this criterion of the device (10). Preferably, the deviation between one device and another, and one container and another may vary from 1 to 15%. This feature is important in a therapeutic setting since it ensures a low variability in the dose administered for a standardized puff, and thus to the delivery of a constant dose. Indeed, this feature combined with the composition according to the invention complies favorably with the concept of vaporization according to the invention.

Another feature is the planar geometry of the heater (34). Heaters (34) manufactured in a flat shape allow easily to automate the manufacturing. Heaters (34) can be in a serpentine or in a mesh shape, but remain in a planar volume. Said shapes can be obtained from a sheet of material (e.g. steel), through cutting or punching. According to the invention, the mesh heater is only usable because the distillate is diluted in the diol solvent. Indeed, the distillate alone is too viscous and would burn the mesh heater if vaporized as it is, i.e. without dilution. The heaters can also be made of ceramic.

Moreover, a flat design of the heater (34) allows a good contact against the absorbing barrier (22), as only geometrical tolerances are critical to ensure the contact quality between the heater (34) and the absorbing barrier (22). Cylindrical shapes for the heater (34) and the absorbing barrier (22) make it more difficult to comply with both dimensional and geometrical tolerances.

Another feature is the low power consumption of the heater (34). In order to control the behavior of the heater (34) over time, the power delivered is limited to a low level, for example equal or below 25W, preferably ranging from 6 W to 15 W or even more preferably ranging from 6W to 10W.

A low power supply implies a slower discharge of the power supply (31).

By consequence, a difference in the load level of the power supply (31) between:
- an initial state, wherein the vaporizing device (10) is new, and
- a final state, wherein the vaporizing device (10) has a nearly empty container (21), will be smaller.

This is advantageous because a smaller difference in the load level implies a smaller difference in the amount of energy the power supply (31) is able to provide, between the initial state and the final state. This ensures a nearly identical behavior of the heater (34), no matter the initial state or the final state of the vaporizing device (10). Even when the power supply (31) is only loaded to 80%, sufficient quantity of vapor is still generated for every puff.

Another feature for ensuring a constant delivery resides in the precise quantity of vapor generated per puff: power supply and/or air flowrate can be adapted for this purpose.

Firstly, the controller (32) is preferably configured to activate the energization during a predetermined duration. By doing so, each puff comprises the same volume of vaporized e-liquid. The predetermined duration can be 3 seconds, or up to 5 or 6 seconds. In a preferred embodiment, the predetermined duration is set to 6 seconds, afterwards the device (10) stops the vaporization.

But the breathable dose, even if highly dependent on the puff duration, also depends on the flow rate of breathed in air.

By consequence, a puff activator of device (10), which leads the controller (32) to energize the heater (34), is preferably a flow sensor (33) rather than a button placed on the housing. A button could be pressed whereas the user is not yet breathing in the vaporized liquid from the duct (11), resulting in losses of vapor, or in a re-deposition of the vapor on surfaces of the device (10). A flow sensor (33) ensures the vaporized liquid will be inhaled by the user.

The flow sensor (33) can be configured to detect when air is breathed in, for example by detecting a depression resulting from the user suction through the duct (11), or by detecting a flow rate of air traversing the duct (11).

Notably, the controller (32) can be configured to energize the heater (34) only if the depression caused by the user suction is above a predetermined threshold, thus ensuring the vaporized e-liquid will reach rapidly the lungs of the user, avoiding a too important re-adsorption of the cannabinoids, for example onto the mouth of the user. Such re-adsorption would lead to imprecisions of the quantity of compounds really delivered to the user.

Then in order to ensure the breathing flowrate is the most constant as possible, from a user to another, or from a puff to another, the duct (11) can be sized or can comprise mechanical restrains in order to limit the breathing in flow rate to levels comprised between 1 L/min to 3 L/min. This way, the influence of respiratory parameters on the airflow are reduced.

Instead of being generally rectilinear, the duct (11) can also comprise a V-shape, wherein heater (34) is located at the tip of the V-shape. In that case, the air (a) breathed inside the vaporizing device (10):
- comes into the duct (11) through a first branch of the V-shape,
- then collides with the heater (34) and loads itself with vaporized e-liquid,
- before having to change its direction and rise again in the second branch of the V-shape.

This particular change of direction upon mixing with the vapor adds inertia to the flow, ensuring a lot of vapor can be generated, even with a little power supplied to the heater (34). By consequence, the V-shape also helps to reduce the power needed to be supplied to the heater (34), which, again, helps to maintain a constant vapor generation per puff whatever is the use level of the device (10).

Other shapes of the duct (11) can be feasible with the same result (e.g. U-shape, or a change of section of the duct (11)), the criterion being the change of speed of the air inside the duct (11).

Lastly, the composition according to the invention can have an impact on the durability of the heater (34). If too many impurities are present in the e-liquid, said impurities may deposit on the heater (34) and prevent a correct heat transfer between the heater (34) and the e-liquid. The same problem may occur when the viscosity of the composition is too high. Due to the bad heat transfer, the heater (34) overheats, which can lead to a degradation of the e-liquid components, and/or to a failure of the heater (34) itself.

The composition according to the invention comprises a distillate (1) of at least one cannabinoid selected from THC, CBD and their mixtures thereof,
wherein said distillate comprises less than 1% by weight of terpene, based on the total weight of distillate, and (2) a diol. The distillate of the present invention is preferably obtained by a molecular distillation that, as already explained above, results in a pure composition, which is even purer after dilution with the diol solvent.

### The kit

The invention also relates to a kit comprising:
(a) a liquid and vaporizable composition comprising
   (1) a distillate of at least one cannabinoid selected from THC, CBD and their mixtures thereof, wherein said distillate comprises less than 1% by weight of terpene, based on the total weight of distillate, and
   (2) a diol selected from 1,2-propanediol 1,3-propanediol, 1,3-butanediol, 1,4-butanediol, 2-methyl-1,3-propanediol, polyethylene glycol (PEG), polyethylene glycol 400 (PEG 400), and combinations thereof; the content of diols ranging from 80 to 98,5% by weight, based on the total weight of the composition; and
(b) a vaporizing device able to deliver a constant dose of cannabinoid(s), ranging from 50 µg to 700 µg per puff and/or per breathable dose.

According to an embodiment, the invention concerns a process of cannabis withdrawal using the kit according to the invention. Such a process comprises the stepwise intake of the composition according to the invention, comprising decreasing concentration of THC and increasing the concentration of CBD.

According to this embodiment, the invention refers to a process of cannabis withdrawal comprising the stepwise intake of the composition according to the invention, comprising decreasing concentration of THC ranging from 25 mg/ml to 5 mg/ml, preferably 25, 20, 15, 10, 5 mg/ml.

Still according to this embodiment, the invention refers to a process of cannabis withdrawal comprising the stepwise intake of the composition according to the invention, comprising increasing concentration of CBD ranging from 5 mg/ml to 25 mg/ml, preferably 5, 10, 15, 20, 25 mg/ml.

According to an alternative embodiment, the invention refers to the composition according to the invention for use in cannabis withdrawal comprising the stepwise intake of the composition according to the invention, comprising decreasing concentration of THC and increasing the concentration of CBD,
wherein the decreasing concentration of THC ranges from 25 mg/ml to 5 mg/ml, preferably 25, 20, 15, 10, 5 mg/ml, and/or
the increasing concentration of CBD ranges from 5 mg/ml to 25 mg/ml, preferably 5, 10, 15, 20, 25 mg/ml.

As an example, a process of cannabis withdrawal can comprise the following stages:
a. Stage #1: THC at a concentration of at least 20mg/ml This is the starting level for heavy cannabis smokers who initially need a highly concentrated THC product to help them compensate for cannabis withdrawal and the associated THC deficiency. In this first stage, there is no cannabidiol (CBD): the focus is on THC substitution for cannabis dependence.
b. Stage #2: THC at a concentration comprised between 15mg/ml and 20 mg/ml + CBD at a concentration comprised between 5mg/ml and *10mg*/*ml.*
c. Stage #3: THC at a concentration comprised between 10mg/ml and 15mg/ml + CBD at a concentration comprised between 10mg/ml and 15 mg/ml*.*
   Stage #2 and #3 are for smoking cannabis and THC withdrawal. They enable the more or less moderate smokers to begin the withdrawal of smoking cannabis via THC substitution at concentrations that remain high, while starting CBD intake via low concentrations enabling the body to begin assimilating this molecule gently, while working on THC dependence and compensating for craving and anxiety linked to THC reduction.
d. Stage #4: THC at a concentration comprised between 5mg/ml and 10mg/ml + CBD at a concentration comprised between 15mg/ml and 20 mg/ml
   Once the subject has passed the first stage, he/she can change stages every 15 days, provided he/she doesn't smoke cannabis during this transition period. If he/she does smoke cannabis, it is advisable to start again at the lower level and begin a new 15-day period at this level.
   In stage #4, THC is no longer predominant in the composition, work is done on THC dependence and anxiety linked to cannabis withdrawal is partly compensated by CBD.
e. Stage #5: THC-free (0 mg/ml) + CBD at a concentration of at least 25mg/ml.
   At this stage, consumption should naturally have decreased, and the frequency of vaporizing device use should be reduced. The CBD present compensates for the drop in THC.
f. Stage #6: Vaporizing device withdrawal stage
   At this stage, the aim is to be THC-free, with vaporizing device use restricted to the end of the day or evening. This level focuses on vaping withdrawal. With the exclusive use of CBD, the non-addictive molecule in high concentration, the aim is to break the memory bridges linking users to their attachment to cannabis and the associated notion of pleasure. After a few weeks at this final stage, the aim is to be able to stop smoking altogether, and stop using a vaporizing device used for cannabis withdrawal.
g. Change stages every 3 weeks if no relapse, otherwise return to the previous stage. A maximum of 6 weeks on one stage would be envisaged to remain in medical and not recreational use.

The invention finally relates to a vaporizing device comprising the liquid and vaporizable composition according to the invention able to deliver a constant dose of cannabinoid(s), ranging from 50 µg to 700 µg per puff and/or per breathable dose.

The inventors have found that the combination of the composition according to the invention, with the vaporizing device, having preferably a mesh heater, acts in synergy to solve the technical problems by delivering a constant dose of the active ingredients to the subject in need thereof. The diluted distillate in the diol solvent is the first important feature that will lead to the delivery of a constant dose; indeed the distillate alone is too viscous and it would not be possible to deliver the active ingredients in an aerosolized and have a good breathable dose. The second important feature of the composition is the boiling points of the ingredients that vary from 180°C to 240°, which means that they share very close boiling points, that permits the vaporization of the active ingredients to be at the same temperature as the diol solvent which optimizes the breathable dose and thus the constant dose delivery. All these features together added to the vaporizing device settled at a predetermined duration of puff intake, and optionally the mesh heater that optimizes the breathable dose due to its better reactivity with the composition, permits the delivery of a constant dose of the active ingredients for use as a medicament.

### BRIEF DESCRIPTION OF THE DRAWINGS

**Figure 1** illustrates the particle size profile of form.1 THC 20 mg/mL expressed in terms of median aerodynamic diameter by mass of THC (MAD). All five replications are represented.
**Figure 2** illustrates the particle size profile of form.2 THC and CBD at 20 mg/mL expressed in terms of median aerodynamic diameter by mass of THC (MAD). The three replications are represented.
**Figure 3** illustrates the particle size profile of form.2 THC and CBD at 20 mg/mL expressed in terms of median aerodynamic diameter by mass of CBD (MAD). The three replications are represented.
**Figure 4** shows the average particle size profile of the form.1 THC 20 mg/mL (round shape), the form.2 THC 20 mg/mL (diamond-shaped) and the form.2 CBD 20 mg/mL (square shape) expressed in terms of median aerodynamic diameter by mass (MAD). The results are presented as an average of five (Form.1) and three (Form.2) independent experiments ± standard deviation (SD).
**Figures 5** is a histogram that shows the median aerodynamic diameter by mass (MAD). The results are presented as an average of five (Form.1) and three (Form.2) independent experiments ± standard deviation (SD). NS: Non-significant. ANOVA with multiple comparisons.
**Figure 6** is a schematic illustrating the structure of a vaporizing device according to the invention.
**Figure 7** is a photo representing a formulation comprising a THC extract (left - comparative formulation B) and a formulation comprising a distillate comprising THC (right - formulation A according to the invention), wherein the extract and the distillate have been diluted in 1,3-propanediol.

### EXAMPLES

The present invention is further illustrated by the following examples.

### Example 1: THC distillate production

First step is grinding dried Cannabis sativa flowers with a humidity level of 10% using a universal cutting mill (Pulverisette 19, Fritsch) at 1800 rpm and tamis 4mm. At this step, the total content of THC is 18,16%, with 18,83% of THCa and 1,66% THC.

Second step is decarboxylation. Heating the flowers in a thermostatic chamber to convert the acidic forms of cannabinoids, THCa, into neutral forms, THC during 90 min at a temperature of 110°C. After this step the total content of THC is 20,30% with 0,14% of THCa and 20,18% of THC.

Third step is extraction using supercritical CO2 as explained in table 1.

**Table 1:**

| **Step 3** | **Supercritical CO₂ extraction** | | | | | |
|---|---|---|---|---|---|---|
| **Process conditions** | | **Quantity of flowers used (g)** | **Recovered soft extract mass (g)** | **THC content (%)** | | |
| | | | | **THCₐ** | **THC** | **THCₜₒₜ** |
| 150 bar / 40°C 100 kg/h solvent-to-solute ratio: 300 kg/kg eq. THC | | 9730 | 2022 | 0,31 | 74,66 | 74,94 |

Fourth step is molecular distillation as explained in table 2.

Equipment: short-path wiped film distillation, evaporator surface 0,05 m².

Molecular distillation in two steps:
a. First step to remove traces of water and volatile terpenes (condensate is recovered)
b. Second step distillation of condensate to remove heavy fractions (e.g. waxes), the distillate is recovered.

**Table 2:**

| **Process conditions** | | **Quantity used (g)** | **Recovered quantity (g)** | **THC content (%)** | | |
|---|---|---|---|---|---|---|
| | | | | **THCa** | **THC** | **THCₜₒₜ** |
| Step 1 Heating Temperature: | Step 2 Heating Temperature: | 495,0 | 335,1 | 0,12 | 83,87 | 83,98 |
| 70°C | 70°C | | | | | |
| Tₑᵥₐₚ: 120°C | Tₑᵥₐₚ: 160°C | | | | | |
| T_{cond}: 60°C | T_{cond}: 80°C | | | | | |
| Pressure: 0,45 mbar | Pressure: 10.10⁻³ mbar | | | | | |
| Total time: 4 h | | | | | | |

| | | | | | | |
|---|---|---|---|---|---|---|
| Tₑᵥₐₚ = evaporator surface temperature T_{cond} = condenser temperature | | | | | | |

Final step is ethanol pre-treatment for filtration, preferably the distillate is dissolved in EtOH (extract/EtOH ratio 1/5 m/m), stored at -18°C, cold filtered and evaporated under vacuum to remove EtOH (cf. table 3)

**Table 3:**

| **Step 5** | **Glaçage** | | | | | |
|---|---|---|---|---|---|---|
| **Process conditions** | | **Quantity used (g)** | **Recovered quantity (g)** | **THC content (%)** | | |
| | | | | **THCa** | **THC** | **THCₜₒₜ** |
| 72H at -18°C then filtration/evaporation | | 76,0 | 66,7 | 0,06 | 81,79 | 81,84 |

**Table 4: Cannabinoids profile of THC-dominant Cannabis sativa extract SCL-4036-F01-C1/1**

| **cannabinoids** | **CBDV** | **CBDa** | **THCV** | **CBD** |
|---|---|---|---|---|
| % m/m | <LOD | <LOD | 0,69 | 0,7 |
| CV % | NA | NA | 1,8 | 0,4 |

| **cannabinoids** | **CBDa2** | **CBG** | **THCVa** | **CBN** |
|---|---|---|---|---|
| % m/m | <LOD | 5,39 | <LOD | 0,77 |
| CV % | NA | 0,9 | NA | 0,8 |

| **cannabinoids** | **CBGa** | **THC9** | **THC8** | **CBL** |
|---|---|---|---|---|
| % m/m | 0,17 | 81,79 | 0,11 | <LOD |
| CV % | 4,1 | 1,1 | 8,5 | NA |

| **cannabinoids** | **CBNa** | **CBC** | **THCa** | **CBCa** |
|---|---|---|---|---|
| % m/m | <LOD | 1,44 | 0,06 | <LOD |
| CV % | NA | 0,3 | 7 | NA |

| **cannabinoids** | **CBD total** | **THC total** | | **CBG total** |
|---|---|---|---|---|
| % m/m | 0,7 | 81,84 | | 5,54 |

**Table 5: Terpenes content in mg/g of distillate**

| **Terpenes** | **Alpha-Pinene** | **Camphene** | **beta-Myrcene** | **(-)-beta-Pinene** | **delta-3-Carene** |
|---|---|---|---|---|---|
| *mg*/*g* | <LOD | <LOD | <LOD | <LOD | <LOD |
| CV% | *NA* | *NA* | *NA* | *NA* | *NA* |

| **Terpenes** | **alpha-Terpinene** | **Ocimene 1** | **d-Limonene** | **p-Cymene** | **Ocimene 2** |
|---|---|---|---|---|---|
| *mg*/*g* | <LOD | <LOD | <LOD | <LOD | <LOD |
| CV% | *NA* | *NA* | *NA* | *NA* | *NA* |

| **Terpenes** | **gamma-Terpinene** | **Terpinolene** | **Linalool** | **(-)-Isopulegol** | **Geraniol** |
|---|---|---|---|---|---|
| % m/m | <LOD | <LOD | <LOD | <LOD | <LOD |
| CV % | *NA* | *NA* | *NA* | *NA* | *NA* |

| **Terpenes** | **beta-Caryophyllene** | **alpha-Humulene** | **Nerolidol 1** | **Nerolidol 2** | **(-)-Guaiol** |
|---|---|---|---|---|---|
| *mg*/*g* | 0,0266 | 0,0125 | 0,0069 | 0,0071 | <LOD |
| CV % | *6,8* | *5,8* | *1,4* | *6,1* | *NA* |

| **Terpenes** | **(-)-alpha-Bisabolol** | | | | |
|---|---|---|---|---|---|
| *mg*/*g* | 0,0096 | | | | |
| CV % | *6,5* | | | | |

| | | | | | |
|---|---|---|---|---|---|
| LOD (Limit of Detection): 0.003 mg/g LOQ (Limit of Quantification): 0.005 mg/g | | | | | |

The highest value is for b-caryophyllene with 0.027 mg/g in the distillate. With a 1:20 dilution in the solvent, it drops to 0.0014 mg/g or 1.4 ppm.

### Conclusion

In conclusion, the above distillation method results in a distillate comprising a terpene-free THC composition which facilitates vaporization of the composition, the 18,16% representing minor cannabinoids. The content of terpenes is less than 1% by weight of the distillate.

### Example 2: Importance of molecular distillation step

A formulation A according to the present invention and a comparative formulation B have been assessed:
- formulation A: a distillate (which corresponds to an extract that has been winterized and has been purified by molecular distillation) diluted in 1,3-propanediol.
- formulation B: an extract (which has been winterized) diluted in 1,3-propanediol.

Winterization process is a purification process used during extraction and refinement that creates pure, potent, and safe cannabis extracts. The extract comprises THC and a ballast. The ballast refers to the residual compounds present in a cannabinoid preparation. These may include waxes, lipids, chlorophylls or other impurities left over from the extraction of the cannabis plant. These residues are eliminated during the distillation process to obtain a purer distillate.

The first target that has been defined was a THC content of 38 mg/mL to reach 0.25 mg per puff. In this experiment, 0.25 mg per puff is the minimum dose per puff. (It has been hypothetically assumed that THC and CBD exhibit identical behavior.)

### Results:

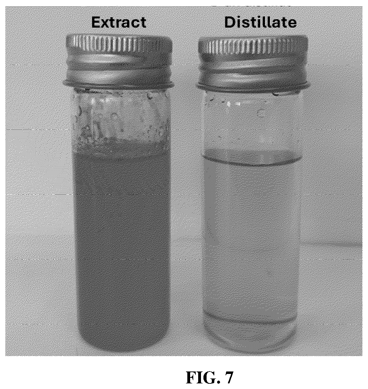

As shown in figure 7, the comparative formulation B, which comprises an extract, is not soluble in the solvent, whereas formula A, which comprises a distillate, is soluble in the solvent (cf. figure 7, left).

The THC concentration measured with HPLC is about 38 mg/mL in both formulations, comprising either the extract (B) or the distillate (A). These results clearly show that the THC is soluble but not part of the ballast present in the extract of formulation B. Indeed, as observed in figure 7, formulation A (which comprises the distillate) is clearer than formulation B (which comprises the extract) since it does not contain the ballast thanks to molecular distillation step.

The results show the importance of the distillation step to obtain a distillate that will lead to an optimal dissolution in 1,3-propanediol.

### Example 3: Performance assessment of an electronic cigarette device in terms of granulometry and respirable dose for cannabinoid delivery.

### Materials and Methods

The proposed experimental approach consists of two distinct phases:
a. Characterization of active ingredient aerosol granulometry by determining the mass median aerodynamic diameter using a Next Generation Impactor (NGI)-type cascade impactor, recognized by the European Pharmacopoeia. The Next Generation Impactor (NGI) is a cascade impactor designed for measuring aerosol particle size distributions.
b. Characterization of active ingredient aerosol dosimetry by determining respirable and non-respirable doses using a Gehling-Type Inhalation (GTI)-type device, which is recognized by the European Pharmacopoeia. These devices are designed to study how aerosols deposit in the respiratory tract, which is crucial for pharmaceutical inhalers, toxicology studies, and environmental exposure assessments.

### Aerosol dosimetry : determination of breathable and non-breathable doses using a GTI-type device

Two cannabinoids were tested, Tetrahydrocannabinol (THC) and Cannabidiol (CBD) using two formulations:
a. **Formulation 1 (Form.1)** : 100% THC at 20 mg/mL
b. **Formulation 2 (Form.2)** : Mixture of THC et CBD at a concentration of 20 mg/mL each.

1 thermal aerosol generator has been used for this experiment.
5 measurements for Form.1 (n=5) and 3 measurements for Form.2 (n=3) have been done.

The respirable and non-respirable doses have been determined using a GTI-type device. (Ambient temperature: 24.55 ± 2.15°C, Relative humidity: 42 ± 1%).
a. Constant flow rate set at 60 ± 0.5 L/min (checked and calibrated at each test).
b. Aerosolization of two sets of 20 puffs in the GTI generated from an automated instrumented syringe (PE_MOD, Burghart, Germany) according to AFNOR XPD-90-300-3 (3-second puff and 55 mL with a 30-second interpuff interval). 300 seconds of waiting between the two series.
c. Collection of puffs in 7 mL of butanol for the upper chamber of the GTI (non-respirable dose) and in 30 mL of butanol for the lower chamber of the GTI (respirable dose)
d. Labeling and storage of suspensions for dosing.

### Results

**Table 6: Aerosol masses generated per puff (mg) for the two formulations tested**

| **Formulation** | **replications** | **Aerosol mass generated (mg/puff)** | | |
|---|---|---|---|---|
| | | **Experimental value** | **Average** | **standard deviation** |
| **Form1. 100% THC (20 mg/mL)** | n=1 | 6,90 | 6,84 | 0,21 |
| | n=2 | 7.00 | | |
| | n=3 | 6,70 | | |
| | n=4 | 7,05 | | |
| | n=5 | 6,55 | | |
| **Form2. Mixture of THC and CBD (20 mg/mL)** | n=1 | 6,68 | 6,74 | 0,23 |
| | n=2 | 7.00 | | |
| | n=3 | 6,54 | | |

The two formulations tested generated an identical average aerosol mass per puff (6.84 mg/puff for form.1 and 6.74 mg/puff for form.2; difference not statistically significant). The composition of the formulation (THC alone, or the combination of CBD with THC) does not change the aerosol mass generated. This result allows reliable comparisons for emitted dose tests between the two formulations. In addition, the very low standard deviation underlines a high inter-puff repeatability for both formulations, demonstrating an excellent performance on this criterion of the device.

These results show that the composition according to the present invention ensures a low variability in the dose administered for a standardized puff, with a fixed volume and duration, and thus a constant delivery dose of the composition according to the invention using a vaporizing device.

**Table 7: Amounts of canabinoids collected depending on the formulation (Form 1 THC at 20 mg/mL or Form 2 CBD and THC mixture at 20 mg/mL, respectively) and distribution between the different respirable and non-respirable fractions (µg/puff).**

| **Formulation** | | **replications** | **Non-breathable dose (µg/puff)** | | | **breathable dose (µg/puff)** | | |
|---|---|---|---|---|---|---|---|---|
| | | | **Experimental value** | **Average** | **standard deviation** | **Experimental value** | **Average** | **standard deviation** |
| **Form1. 100% THC (20 mg/mL)** | | n=1 | 0,16 | 0,15 | 0,09 | 72,32 | 75,20 | 10,73 |
| | | n=2 | 0,25 | | | 70,30 | | |
| | | n=3 | 0,17 | | | 73,97 | | |
| | | n=4 | 0,00 | | | 93,60 | | |
| | | n=5 | 0,17 | | | 65,80 | | |
| **Form2. Mixture of THC and CBD (20 mg/mL)** | **THC dosage** | n=1 | 0,14 | 0,26 | 0,12 | 65,22 | 64,39 | 4,59 |
| | | n=2 | 0,39 | | | 59,44 | | |
| | | n=3 | 0,26 | | | 68,50 | | |
| | **CBD dosage** | n=1 | 0,00 | 0,00 | 0,00 | 68,89 | 67,61 | 6,60 |
| | | n=2 | 0,00 | | | 60,47 | | |
| | | n=3 | 0,00 | | | 73,48 | | |

The breathable doses of THC collected range from 64.39 µg/puff (Form.2 THC and CBD at 20 mg/mL) to 75.20 µg/puff (Form.1 THC at 20 mg/mL). Non-breathable doses of THC range from 0.15 µg/puff (Form.1 THC at 20 mg/mL) to 0.26 µg/puff (Form.2 THC and CBD at 20 mg/mL). These results therefore demonstrate that the vaporizing device is able to generate a breathable dose containing THC at more than 99.5% wt : 99.8%wt for formulation 1, and 99.6%wt for formulation 2.

The approximately 15% decrease in the amount of THC collected in respirable doses between Form.1 and Form.2 is statistically non-significant.

The same respirable dose values for THC for both formulations containing the same nominal THC concentration are found, i.e., the potential presence of CBD in the formulation has no impact on the respirable dose of THC emitted.

The average amounts of CBD collected were 64.61 µg/puff (Form.2 THC and CBD 20 mg/mL) for the breathable dose, and 0.00 µg/puff (Form.2 THC and CBD 20 mg/mL) for the non-breathable dose. Therefore, the vaporizing device can generate a breathable dose containing 100%wt of CBD. The breathable amounts of THC and CBD in Formulation 2 are identical, and therefore directly correlated with their identical concentration of 20 mg/mL in the formulation.

Finally, it is important to note that the amounts of cannabinoids collected in the GTI (breathable dose + non-breathable dose) are lower than the theoretical aerosolization dose. This theoretical dose is calculated by considering a transfer efficiency of 100% between the concentration of the cannabinoid in the initial formulation and the mass of aerosol generated (theoretical dose of aerosolization = [cannabinoid] in the formulation x mass of aerosol generated). Thus, the theoretical dose of THC was calculated at 132.80 and 129.01 µg/puff, respectively for formulations 1 and 2. Similarly, the theoretical dose of CBD was calculated at 134.75 µg/puff for formulation 2. These results allow calculating a transfer efficiency of cannabinoids in the aerosol phase from the nominal concentration in the initial formulation, which is between 50.03% and 56.54%.

**Table 8: Distribution of respirable and non-respirable dose of the active ingredients of each formulation (% of nominal dose)**

| **Formulation** | | **replications** | **Non-breathable dose (% of the nominal dose)** | | | **breathable dose (% of the nominal dose)** | | |
|---|---|---|---|---|---|---|---|---|
| | | | **Experimental value** | **Average** | **standard deviation** | **Experimental value** | **Average** | **standard deviation** |
| **Form1. 100% THC (20 mg/mL)** | | n=1 | 0,12 | 0,11 | 0,06 | 53,99 | 56,54 | 6,94 |
| | | n=2 | 0,18 | | | 51,72 | | |
| | | n=3 | 0,13 | | | 56,87 | | |
| | | n=4 | 0,00 | | | 68,38 | | |
| | | n=5 | 0,13 | | | 51,74 | | |
| **Form2. Mixture of THC and CBD (20 mg/mL)** | **THC dosage** | n=1 | 0,11 | 0,20 | 0,09 | 50,99 | 50,03 | 5,22 |
| | | n=2 | 0,29 | | | 44,38 | | |
| | | n=3 | 0,20 | | | 54,71 | | |
| | **CBD dosage** | n=1 | 0,00 | 0,00 | 0,00 | 51,57 | 50,33 | 6,57 |
| | | n=2 | 0,00 | | | 43,23 | | |
| | | n=3 | 0,00 | | | 56,19 | | |

The dosimetry data (percentage of the nominal dose) considers the concentration of the active ingredient in the initial formulation. Therefore, it becomes possible to normalize the distribution of the cannabinoids of respirable and non-respirable dose by showing the transfer efficiency of the active substance present in the formulation to the particle phase of the aerosol.

Following up the above conclusions, about 50±5%wt of the nominal dose of THC and CBD is present in the respirable fraction of the aerosol generated. Furthermore, the non-respirable fraction represents less than 0.2±0.1%wt of the nominal dose for the two formulations tested. This result highlights that the performance of the thermal aerosol generation device is at least equivalent to, or even superior to, the nebulizers usually used in clinical practice for the administration of active ingredients such as bronchodilators, where the respirable dose is generally between 20 and 40% of the nominal dose. These results show that the tested device is therefore capable of generating aerosols with an aerodynamic diameter of less than 6.4µm containing the composition according to the invention with more than 50%wt of the nominal dose.

### Conclusion

The results of this study made it possible to characterize the different parameters, dosimetry and particle size, in a similar way to the requirements of Annex CC of the AFNOR 13544-1 standard of November 2009 for nebulizers, while adapting the experimental protocols in order to be relevant to the specificities of this thermal aerosol generation device.

The thermal aerosol generation device shows a high reproducibility in the amount of aerosol generated per puff. This intrinsic characteristic is fundamental for the administration of active ingredients. The formulation, THC alone or THC+CBD mixture, has no impact on the doses of THC and CBD emitted in the aerosol. Furthermore, more than 99.5%wt of aerosolized cannabinoids are present in the particle size fraction corresponding to the respirable dose (diameters < 6.4µm). More than 50%wt of cannabinoids in relation to the nominal dose are present in the particle size fraction corresponding to the respirable dose (diameters < 6.4µm). In other words, more than 50% of cannabinoids present in the initial dose of the composition according to the invention will be bioavailable to the subject in need.

### Aerosol granulometry: determination of mass median aerodynamic diameter using an NGI cascade impactor

Two cannabinoids were tested, Tetrahydrocannabinol (THC) and Cannabidiol (CBD) using two formulations:
a. **Formulation 1 (Form.1)** : 100% THC at 20 mg/mL
b. **Formulation 2 (Form.2)** : Mixture of THC et CBD at a concentration of 20 mg/mL each.

1 thermal aerosol generator has been used for this experiment.
5 measurements for Form.1 (n=5) and 3 measurements for Form.2 (n=3) have been done.

Determination of the median aerodynamic diameter by mass using an NGI type cascade impactor (Ambient temperature: 22.5 ± 1.66°C, Relative humidity: 43.33 ± 1.77%).
a. Constant flow rate set at 60 ± 0.5 L/min (calibrated and checked with each test).
b. Aerosolization of 20 puffs in the GTI generated from an automated instrumented syringe (PE_MOD, Burghart, Germany) according to AFNOR XPD-90-300-3 (3-second puff and 55 mL with a 30-second interval between puffs)
c. Collection of puffs on each NGI tray with 2 mL of absolute ethanol
d. Labeling and storage of suspensions for dosing.

### Results

**Table 9: Particle size data expressed in terms of median aerodynamic diameter by mass (MAD) and geometric standard deviation (GSD).**

| **Formulation** | | **replications** | **MAD (µm)** | **Average MAD (µm)** | **MAD standard deviation** | **GSD** | **Average GSD** | **GSD standard deviation** |
|---|---|---|---|---|---|---|---|---|
| **Form1. 100% THC (20 mg/mL)** | | n=1 | 0,86 | 0,90 | 0,03 | 1,60 | 1,60 | 0,01 |
| | | n=2 | 0,93 | | | 1,61 | | |
| | | n=3 | 0,88 | | | 1,60 | | |
| | | n=4 | 0,94 | | | 1,93 | | |
| | | n=5 | 0,92 | | | 1,58 | | |
| **Form2. Mixture of THC and CBD (20 mg/mL)** | **THC dosage** | n=1 | 0,90 | 0,91 | 0,04 | 1,61 | 1,60 | 0,00 |
| | | n=2 | 0,96 | | | 1,61 | | |
| | | n=3 | 0,86 | | | 1,60 | | |
| | **CBD dosage** | n=1 | 0,89 | 0,90 | 0,04 | 1,61 | 1,60 | 0,00 |
| | | n=2 | 0,95 | | | 1,61 | | |
| | | n=3 | 0,86 | | | 1,60 | | |

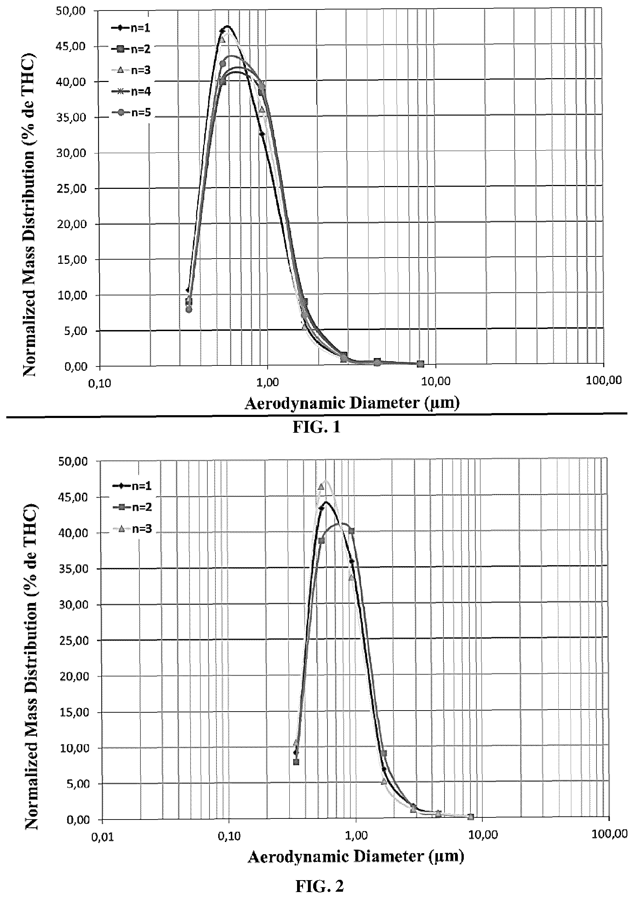

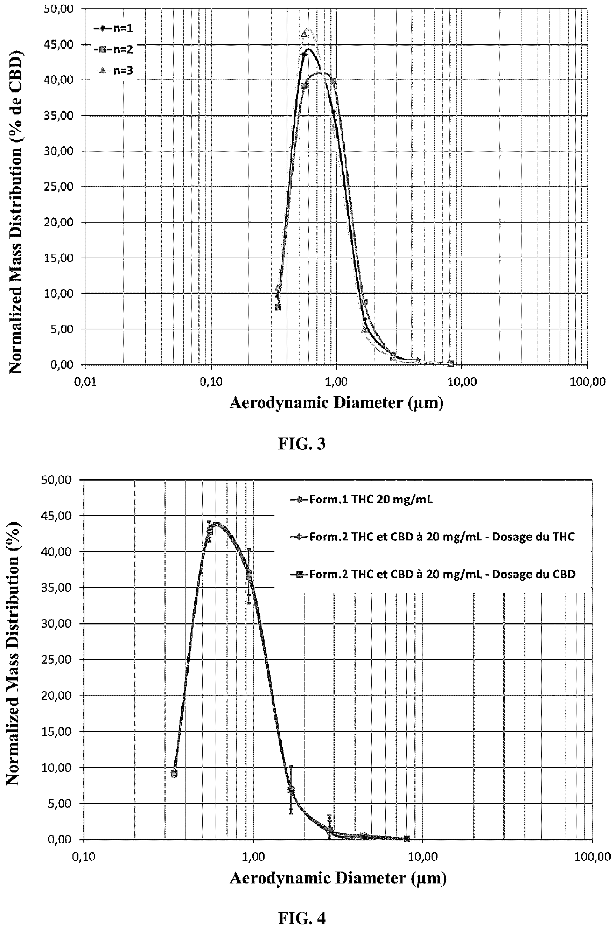

The resulting curves of figures 1 to 4 illustrate the distribution of the normalized aerodynamic mass as a function of the aerodynamic diameter. Figures 1, 2 and 3 represent the full replicates performed for the form.1 THC 20 mg/mL, form.2 THC and CBD at 20 mg/mL by mass of THC and CBD, respectively. Figure 4 compiles the replicates and shows the average of the aerodynamic weight distribution for the three experimental conditions. The aerodynamic diameter (µm) is reported on the x-axis (x) while the y-axis represents the normalized percentage distribution of mass. The Gaussian shapes of the curves indicate symmetrical distribution of the aerodynamic particles measured for each formulation. The particles generated have mainly aerodynamic diameters ranging from 0.5 and 1 µm. The superposition of the curves suggests excellent repeatability in the distribution of particle sizes for each formulation and each cannabinoid studied.

The histogram presented in Figure 5 provides a clear view of the Median Aerodynamic Diameter by Mass (MAD) for the three experimental conditions and reflects the high reproducibility of the device observed on the particle size curves. Indeed, MAD is almost identical regardless of the formulation and the active ingredient studied, without any significant difference between the conditions. The submicron MAD (0.90 µm) is particularly suitable for deep pulmonary deposition of the aerosol compared to clinical nebulizers (generally between 3 and 6 µm) generating particles of a larger diameter that can lead to a loss of part of the active ingredient in the upper airway.

### Conclusion

The device shows a high reproducibility in the particle size of the aerosols generated. The mean aerodynamic diameter is around 0.90±0.04µm. This result is surprising regarding the conventional aerosol therapy technologies such as nebulizers that generate particles of 3 to 6 µm. This particle size profile of around 0.9µm is relevant for the administration of active ingredients deeply into the lung by limiting the loss in the upper airways but also the loss related to the phenomenon of particle exhalation. These aerodynamic characteristics are in favor of a favorable prediction for pulmonary absorption of aerosolized active ingredients, in other words to the bioavailability of the active ingredients. Indeed, when particles have a size of less than 1 µm, they can stay airborne longer, penetrate deep into the alveoli and enter the bloodstream. Therefore, the therapeutic effect of the CBD and THC comprised in the composition according to the invention will be maximized.

The results obtained for the granulometry and the breathable dose using a vaporizing device for cannabinoid delivery validate the fact that the composition according to the invention delivered through a vaporizing device has effective therapeutic effect and can be used as a medicament, notably for pain management and the treatment of cannabinoid addiction.

## Claims

1. A liquid and vaporizable composition comprising
(1) a distillate of at least one cannabinoid selected from THC, CBD and their mixtures thereof,
wherein said distillate comprises less than 1% by weight of terpene, based on the total weight of distillate, and
(2) at least one diol selected from 1,2-propanediol 1,3-propanediol, 1,3-butanediol, 1,4-butanediol, 2-methyl-1,3-propanediol, polyethylene glycol (PEG), polyethylene glycol 400 (PEG 400), and mixtures thereof; the content of diols ranging from 80 to 98,5% by weight, based on the total weight of the composition,
for use as a medicament, wherein a constant dose of cannabinoid(s), ranging from 50 µg to 700 µg per puff and/or per breathable dose, is delivered to a subject in need through a vaporizing device.

2. The composition according to claim 1, for use in a method of pain management, preferably in the prevention and/or treatment of chronic pain, painful spasticity, pain due to cancer, injury, accident, surgery, inflammation, tissue damage, arthritis, joint pain, pain from infection, gastrointestinal pain, diabetes, diabetes neuropathy, post-shingles neuralgia, neuropathic pain, peripheral neuropathy or multiple sclerosis, more preferably chronic pain or painful spasticity.

3. The composition according to claim 1, for use in the prevention and/or the treatment of cannabis addiction.

4. The composition for use according to any one of the preceding claims, wherein the distillate (1) consists (essentially) of THC or CBD.

5. The composition for use according to any one of the preceding claims, wherein the distillate (1) comprises a mixture of THC and CBD and the weight ratio THC:CBD ranges from 0.1 to 10.

6. The composition for use according to any one of the preceding claims, wherein the content of cannabinoid(s) ranges from 60 to 99% by weight, preferably from 80% to 95% by weight, based on the total weight of the distillate.

7. The composition for use according to any one of the preceding claims, wherein the content of distillate (1) ranges from 1,5% to 10% by weight, based on the total weight of the composition.

8. The composition for use according to any one of the preceding claims, wherein the distillate (1) comprises less than 0,9% by weight of terpene, more preferably less than 0,5% by weight of terpene, even more preferably less than 0,1% by weight of terpene, better still less than 0.05% by weight of terpene,; advantageously less than 0.01% by weight of terpene, more advantageously less than 0.005% by weight of terpene, better still less than 0.001% by weight of terpene, more preferably less than 0.0005% by weight of terpene, and even more advantageously less than 0.0002% by weight of terpene, based on the total weight of distillate.

9. The composition for use according to any one of the preceding claims, wherein the diol (2) is 1,3-propanediol.

10. The composition for use according to any one of the preceding claims, wherein the content of diols (2) ranges from 85 to 98,5% by weight, based on the total weight of the composition, preferably 90 to 98,5%.

11. The composition according to any one of the preceding claims, wherein the constant dose of cannabinoid(s) ranges from 55 µg to 500 µg per puff and/or per breathable dose, preferably from 60 to 300 µg, more preferably from 60 µg to 80 µg.

12. The composition for use according to any one of the preceding claims, wherein the composition provides a subject in need with THC:CBD ranging from 50:0 to 10:50 mg/ml, preferably from 40:0 to 10:40 mg/ml.

13. Kit comprising:
(a) a liquid and vaporizable composition comprising
(1) a distillate of at least one cannabinoid selected from THC, CBD and their mixtures thereof, wherein said distillate comprises less than 1% by weight of terpene, based on the total weight of distillate, and
(2) at least one diol selected from 1,2-propanediol 1,3-propanediol, 1,3-butanediol, 1,4-butanediol, 2-methyl-1,3-propanediol, polyethylene glycol (PEG), polyethylene glycol 400 (PEG 400), and combinations thereof; the content of diols ranging from 80 to 98,5% by weight, based on the total weight of the composition; and
(b) vaporizing device able to deliver a constant dose of cannabinoid(s), ranging from 50 µg to 700 µg per puff and/or per breathable dose.

14. A vaporizing device comprising the liquid and vaporizable composition according to any one of claims 1 to 12 able to deliver a constant dose of cannabinoid(s), ranging from 50 µg to 700 µg per puff and/or per breathable dose.
